# EUROPÄISCHE PATENTSCHRIFT

(11) **EP 2 561 362 B1**
(45) Veröffentlichungstag und Bekanntmachung des Hinweises auf die Patenterteilung: **12.09.2018**
(21) Anmeldenummer: 11731241.3
(22) Anmeldetag: 14.04.2011
(51) Int. Cl.: G01N 33/569

(54) **VERFAHREN ZUR ERKENNUNG EINER SALMONELLENINFEKTION**
METHOD FOR DETECTING A SALMONELLA INFECTION
PROCÉDÉ POUR RECONNAÎTRE UNE INFECTION PAR DES SALMONELLES

(30) Priorität: 23.04.2010 DE 102010018085
(43) Veröffentlichungstag der Anmeldung: 27.02.2013
(73) Patentinhaber: Bioserv Analytik und Medizinprodukte GmbH, 18059 Rostock (DE)
(72) Erfinder: MEYER, Udo, 18239 Hastorf (DE); STEINMANN, Alain, 18057 Rostock (DE); FAHLANDT, Heike, 18276 Bülower Bogen (DE); SCHULZ, Babette, 18057 Rostock (DE)
(74) Vertreter: Baumbach, Friedrich
(86) Internationale Anmeldenummer: PCT/DE2011/000460
(87) Internationale Veröffentlichungsnummer: WO 2011/131178

(56) Entgegenhaltungen:
- WO-A1-97/18225
- WO-A1-2007/016912
- US-A1- 2007 031 906

## Beschreibung

Die Erfindung betrifft ein diagnostisches Verfahren zur Erkennung einer Salmonelleninfektion gemäß Anspruch 1. Anwendungsgebiete sind die Medizin, Veterinärmedizin und verschiedene Zweige der Industrie.
Bei Salmonellen handelt es sich um bewegliche, gramnegative Stäbchen. Taxonomisch werden die Salmonellen durch das Auftreten von Körper (O)- und Geißel (H)-Antigenen differenziert und in einem Ordnungssystem in Serovare eingegliedert und mittels ihrer Seroformel charakterisiert. Derzeit sind etwa 2.400 Salmonellen-Serovare beschrieben. Bedeutung als Erreger von epidemiologischen Erkrankungen besitzen in der Praxis aber lediglich 20-30 Serovare (z.B. S. typhi, S. paratyphi A, B, C sowie eine große Menge von Enteritiserregern).
Salmonellosen beim Menschen werden zum überwiegend Teil durch den Verzehr von infizierten bzw. kontaminierten Lebensmitteln ausgelöst. Der Kontaktübertragung vom Tier auf den Menschen kommt eine geringe Bedeutung zu. Auch eine direkte bzw. indirekte Übertragung von Mensch zu Mensch ist in der Regel ein seltenes Ereignis, kann jedoch als Hospitalinfektion bei disponierten Personen oder bei hygienisch ungünstigen Verhältnissen erfolgen. Primäre Infektionenquellen sind hauptsächlich von Geflügel, Schweinen und Rindern stammende Lebensmittel. Da die Tiere nur selten selbst erkrankt sind, kommt dem Erreger- bzw. Antikörpernachweis auch in der Veterinärmedizin und der Lebensmittelindustrie eine große Bedeutung zu.
Die Infektionsdosis beträgt bei gesunden erwachsenen Menschen 10⁴ bis 10⁶ Keime. Die Inkubationszeit liegt in Abhängigkeit von der Infektionsdosis zwischen 5 und 72 Stunden. Bei einer Enteritis-Salmonellen-Infektion, äußert sich die Erkrankung mit Durchfall, Übelkeit, Erbrechen und leichtem bis mäßigem Fieber. Die Symptome halten meist nur wenige Stunden bis Tage an, können bei alten und geschwächten Personen oder Kindern auch zum Tode führen.
Die Keimausscheidung nach einer Salmonelleninfektion dauert im Mittel 3 bis 6 Wochen, bei Säuglingen aber unter Umständen auch mehrere Monate. In seltenen Fällen kann auch ein chronischer Verlauf auftreten.
Gemäß Infektionsschutzgesetz ist der Verdacht auf bzw. die Erkrankung an akuter infektiöser Gastroenteritis unter bestimmten Umständen meldepflichtig. Der Salmonellennachweis unterliegt immer der Meldepflicht. In der EU existieren zahlreiche Rechtsvorschriften zur Salmonellenbekämpfung (z.B. Rinder-Salmonellen-Verordnung, Hühner-Salmonellen-Verordnung, verschiedene Bestimmungen des Futtermittel- und Lebensmittelrechtes u.a.). Der Schwerpunkt der lebensmittelanalytischen bzw. klinischen Salmonellendiagnostik liegt in der Anzucht der Erreger aus einem Lebensmittel (auch Schlachtteile) oder aus Stuhl- bzw. Kotproben und im serologischen Nachweis bzw. Ausschluss von Salmonellen im Verdachtsfall mittels omni- oder polyvalenten Salmonellen-Suchseren. Üblicherweise kann eine solche Diagnose erst ca. 1 - 2 Tage nach Eingang der Probe im Labor gestellt werden. Für eine gesicherte Aussage, dass eine Salmonelleninfektion vorliegt, werden weitere 2 - 3 Tage benötigt. Dazu werden verdächtige Einzelklone biochemisch mittels bunter Reihe und serologisch weiter charakterisiert. Die serologische Differenzierung erfolgt mit Antiseren gegen O- und H-Antigene im Agglutinationstest nach dem Kaufmann-White Schema. Das bedeutet, dass für den endgültigen Nachweis einer Salmonelleninfektion 3-5 Tage notwendig sind. Insbesondere bei lebensmittelinduzierten Salmonellosen stellt der lange Zeitraum bis zur Diagnose ein großes Problem dar, weil sich zwischenzeitlich weitere Personen infizieren können. Deshalb kommt es bei einem Salmonellenverdacht darauf an, die Infektionsquelle zu lokalisieren und weitere Infektionen zu verhindern. Damit das kurzfristig erfolgen kann, ist eine möglichst frühe Diagnosestellung von grundsätzlicher Bedeutung.
Der serologische Antikörpernachweis wird vor allem in der Veterinärmedizin und Lebensmittelindustrie in Form von ELISA-Systemen eingesetzt. Der Nachweis erfolgt u. a. aus Fleischpresssaft, Blut oder Tupfer-/Kratzschwammproben. Nachteil dieses Verfahrens ist, dass eine Reihe pathogener Salmonellen-Serovare nicht zuverlässig nachgewiesen werden kann.

In der Humanmedizin wird dagegen bei Salmonelleninfektionen der Widal-Agglutinationstest als Ergänzung zum bakteriologischen Erregernachweis eingesetzt. Nachteil dieses Verfahrens ist, dass zum Einen nicht alle Infektionen zur Bildung von Antikörpern gegen O-Antigene führen bzw. Antikörper gegen H-Antigene über Jahre persistieren können. Da Titer gegen O-Antigene oft schon nach wenigen Wochen so weit abgefallen sind, dass ihr Nachweis nicht mehr möglich ist, kann nicht sicher zwischen einer akuten und einer weiter zurückliegenden Infektion unterschieden werden.
Bei der In-vitro Kultivierung von Salmonellen wird von diesen in das Kulturmedium eine ganze Reihe von Proteinen ausgeschieden. Bei einem dieser Proteine handelt es sich um das sogenannte SipC-Protein (Salmonella Invasions Protein), dessen Nukleotid- und Aminosäuresequenz aus Datenbanken verfügbar ist.

Das SipC-Protein gehört zu den Effektorproteinen, die die Invasion der Salmonellen in die Wirtszellen ermöglicht. Die Invasion wird ermöglicht durch Polymerisation und Kondensation von Aktinfilamenten. Dieser Vorgang wird als "Bundling" bezeichnet.
Die Aufnahme der Bakterien erfolgt über Makropinozytose. Dieser geht ein intensiver Signalaustausch zwischen Pathogen und Wirtszelle voraus. Es kommt zu einer Kräuselung der Membran der Wirtszelle, verursacht durch eukaryontische Wachstumsfaktoren. Durch die Kräuselung der Membran entstehen membrangebundene Vakuolen, in die die Salmonellen aufgenommen werden. Unter normalen Bedingungen wird die Membrankräuselung und die damit einhergehende Bakterieninvasion durch Mikrofilament-Inhibitoren, wie z.B. Cytochalasin D unterbunden. Diese Tatsache verdeutlicht die Notwendigkeit der Aktinpolymerisation für die Bakterieninvasion.
Es wurde gezeigt, dass das SipC-Protein allein, ohne die Wirkung anderer Komponenten der Wirtszelle von dieser aufgenommen wird und zum "Bundling" der Aktinfilamente führt.

Beschrieben ist die Verwendung von PCR-Primern und FRET-Hybridisierungssonden gegen das SipC-Gen zum Nachweis von Salmonellen. Die Verwendung von Antikörpern gegen SipC zum immunologischen Nachweis wurde nicht berücksichtigt. Von HAYWARD et al. (1999) und WEIHRAUCH et al. (2002) wird der SipC-Nachweis im Immunoblot unter Verwendung eines polyklonalen Antikörpers beschrieben. Der Nachteil der verwendeten Antiseren liegt in ihrer Kreuzreaktivität. Daher waren sie nicht für einen spezifischen Salmonellennachweis geeignet.
Es gibt auch mehrere Schutzrechte, die den Nachweis von Salmonellen-Infektionen betreffen. In der US Patent Publication 2007/0031906A1, H. Klein et al, wird ein Testsystem und ein Testverfahren offenbart. Es enthält wenigstens einen monoklonalen Antikörper, der mit dem SipC Protein reagiert. Es wird nicht beschrieben, gegen welche Peptidsequenz der beanspruchte Antikörper gerichtet ist. WO 97/18225 offenbart eine Methode zum Nachweis von Salmonellen unter Verwendung von Antikörpern gegen Ssp welches dem SipC entspricht. Dabei offenbart WO 97/18225 nicht, gegen welche Peptidsequenz der beanspruchte Antikörper gerichtet ist.
Das Ziel der Erfindung besteht darin, einen zuverlässigen Test zum Nachweis von Salmonellen zu entwickeln. Ihr liegt die Aufgabe zugrunde, ein Verfahren aufzubauen, welches einen möglichst frühen Nachweis ermöglicht und alle wichtigen Serovare erfasst.
Die Erfindung wird gemäß den Ansprüchen 1-10 realisiert. Basis der Erfindung ist die Verwendung des SipC-Proteins zum Nachweis einer Salmonelleninfektion. Dieses Protein wird beim Stoffwechsel von Salmonellen ausgeschieden. Kernpunkt der Erfindung ist der überraschende Befund, dass im SipC-Protein 5 hochantigene Areale ausgebildet sind. Die Erfindung beruht darauf, dass diese Areale mit Antikörpern bzw. korrespondierenden Nukleinsäuresequenzen nachgewiesen werden können.
In WO/2007/016912 wird das SipC-Protein als ein stark konserviertes Eiweiß beschrieben und darauf verwiesen, dass in allen Salmonellenserovaren die Aminosäuresequenzen des SipC-Proteins nur äußerst geringe Unterschiede aufweisen und bereits zu einem sehr frühen Zeitpunkt die Proteine des Typ HI Sekretionssystems bilden. Grundlage für den auf dieser Basis entwickelten Nachweis des SipC bilden verschiedene monoklonale Antikörper gegen das Protein. Dieses Nachweissystem erlaubt die Beantwortung der Frage, ob eine aktive Salmonelleninfektion im Organismus vorliegt.
Bei der strukturanalytischen Betrachtung der Aminosäuresequenz mittels 3 verschiedener Methoden fiel auf, dass im Protein überraschender Weise 5 hochantigene Areale ausgebildet sind. Identifiziert wurden folgende Aminosäuresequenzen:
1. Der Proteinabschnitt 363-378 mit der Sequenz VASTASDEARESSRKS (SEQ ID NO 1)
2. Der Proteinabschnitt 15-30 mit der Sequenz NNHSVENSSQTASQSV (SEQ ID NO 2)
3. Der Proteinabschnitt 343-357 mit der Sequenz GQYAATQERSEQQIS (SEQ ID NO 3)
4. Der Proteinabschnitt 276-289 mit der Sequenz LGIKDSNKQISPEH (SEQ ID NO 4)
5. Der Proteinabschnitt 246-260 mit der Sequenz LNMKKTGTDATKNLN (SEQ ID NO 5)

Im Gesamtprotein sind die Peptidabschnitte wie folgt angeordnet:

10 20 30 40 50 60
MLISNVGINP AAYLNNHSVE NSSQTASQSV SAKDILNSIG ISSSKVSDLG LSPTLSAPAP
70 80 90 100 110 120
GVLTQTPGTI TSFLKASIQN TDMNQDLNAL ANNVTTKANE VVQTQLREQQ AEVGKFFDIS
130 140 150 160 170 180
GMSSSAVALL AAANTLMLTL NQADSKLSGK LSLVSFDAAK TTASSMMREG MNALSGSISQ
190 200 210 220 230 240
SALQLGITGV GAKLEYKGLQ NERGALKHNA AKIDKLTTES HSIKNVLNGQ NSVKLGAEGV
250 260 270 280 290 300
DSLKSLNMKK TGTDATKNLN DATLKSNAGT SATESLGIKD SNKQISPEHQ AILSKRLESV
310 320 330 340 350 360
ESDIRLEQNT MDMTRIDARK MQMTGDLIMK NSVTVGGIAG ASGQYAATQE RSEQQISQVN
370 380 390 400
NRVASTASDE ARESSRKSTS LIQEMLKTME SINQSKASAL AAIAGNIRA (SEQ ID NO 6)

Wenngleich die Peptide bereits allein eine Antikörperinduktion auslösen, hat es sich erfindungsgemäß als zweckmäßig erwiesen, diese Peptide an übliche Carriersubstanzen wie z.B. Hämocyanin zu binden. Für die Herstellung der polyklonalen Antipeptidantikörper werden Versuchstiere wie Kaninchen, Meerschweinchen, Ziegen, Hühner oder Fische in bekannter Weise mit den Peptiden immunisiert. Für die Herstellung der erfindungsgemäßen monoklonalen Antikörper werden die Peptide in bekannter Weise für die Induktion spezifischer B-Zellen verwendet, die nach Fusionierung mit Myelomzellen Hybridomzellen generieren, die nach bekannten Klonierungsverfahren kultiviert werden und dann die spezifischen monoklonalen Antikörper sezernieren. Es konnte nachgewiesen werden, dass die mono Antikörper hoch spezifisch mit dem SipC-Protein reagieren.

Das SipC-Protein besteht aus einem hydrophoben Mittelteil (Aminosäuren 121 - 199) und zwei hydrophilen Teilen. Der N-terminalen Domäne (Aminosäuren 1 - 120 und der C-terminalen Domäne (Aminosäuren 200 - 409). Da die hydrophilen Teile des Proteins in hohen Konzentrationen unter physiologischen Bedingungen löslich sind, wurden diese und die Sequenz des gesamten Proteins im Vergleich zu den Sequenzen der identifizierten immunogenen Peptide in *E*. *coli* (Stamm BL21, Vektor pET28a) exprimiert. 
10 20 30 40 50 60
MLISNVGINP AAYLNNHSVE NSSQTASQSV SAKDILNSIG ISSSKVSDLG LSPTLSAPAP
70 80 90 100 110 120
GVLTQTPGTI TSFLKASIQN TDMNQDLNAL ANNVTTKANE VVQTQLREQQ AEVGKFFDIS
130 140 150 160 170 180
GMSSSAVALL AAANTLMLTL NQADSKLSGK LSLVSFDAAK TTASSMMREG MNALSGSISQ
190 200 210 220 230 240
SALQLGITGV GAKLEYKGLQ NERGALKHNA AKIDKLTTES HSIKNVLNGQ NSVKLGAEGV
250 260 270 280 290 300
DSLKSLNMKK TGTDATKNLN DATLKSNAGT SATESLGIKD SNKQISPEHQ AILSKRLESV
310 320 330 340 350 360
ESDIRLEQNT MDMTRIDARK MQMTGDLIMK NSVTVGGIAG ASGQYAATQE RSEQQISQVN
370 380 390 400
NRVASTASDE ARESSRKSTS LIQEMLKTME SINQSKASAL AAIAGNIRA (SEQ ID NO 6)
 Nukleinsäuresequenz:

atg tta att agt aat gtg gga ata aat ccc gcc gct tat tta aat aat cat
tct gtt gag aat agt tca cag aca gct tcg caa tcc gtt agc gct aaa gat
att ctg aat agt att ggt att agc agc agt aaa gtc agt gac ctg ggg ttg
agt cct aca ctg agc gcg cct gcg cca ggg gta tta acg caa acc ccc gga
acg atc acg tcc ttt tta aaa gcc agt att caa aat acc gac atg aat cag

gat ttg aat gct ctg gca aat aat gtc acg act aaa gcg aat gag gtt gtg
caa acc cag tta cgc gag cag gca gaa gtc gga aag ttt ttt gat att
agc gga atg tct tcc agt gcc gtt gcg ctg ttg gct gcc gcg aat acg tta
atg ctg acg ttg aac cag gct gat agc aaa ctg tct ggt aag ttg tca tta
gtc agt ttt gat gca gct aaa acg acg gca agc tcc atg atg cgc gaa ggg
atg aat gcg ttg tcc ggt agt att tcc cag agc gcg ctt cag ttg ggg atc
act ggc gtg ggc gcc aaa ctg gaa tat aag ggg ctg cag aat gaa aga ggc
gcg ctt aaa cat aat gcc gcg aag atc gat aaa ctg acc act gaa agc cac
agt att aaa aac gtg ctg aac ggg cag aat agc gtc aaa ctc ggt gct gaa
ggc gtc gat tct ctg aaa tcg tta aat atg aag aaa acc ggt acc gat gcg
acg aaa aat ctt aat gat gcg acg ctt aaa tct aat gcc gga acc agc gcc
acg gaa agt ctg ggt att aaa gac agt aat aaa caa atc tcc cct gaa cat
cag gct att ctg tcg aaa cgt ctt gag tct gtc gaa tcc gat att cgt ctt
gag cag aat acc atg gat atg acc cga atc gat gcg cgc aag atg cag atg
acg ggc gat ctg att atg aag aac tcg gtc acg gtc ggt ggt att gca ggg
gcg tcc ggg cag tac gcc gct act cag gaa cgt tcc gag cag caa att agc
cag gtg aat aac cgg gtt gcc agc acc gca tcg gac gaa gcc cgt gaa agt
tca cgt aaa tcg acc agc ctg att cag gaa atg ctg aaa aca atg gag agc
att aac cag tcg aaa gca tcc gca ctc gct gct atc gca ggc aat att cgc
gct taa (SEQ ID NO 7)

Im Folgenden werden die Einzelheiten des erfindungsgemäßen Verfahrens noch weiter erläutert. Die Erkennung einer Salmonelleninfektion/-kontamination erfolgt in Exkreten von Patienten/Tieren, Schlachtkörpern, Eiern und Lebens-/Futtermitteln, wobei das SipC aller bekannten Salmonellen-Serovare gemäß Anspruch 1 nachgewiesen wird.

Diese Bestimmung erfolgt mittels immunchemischer Systeme unter Nutzung monoklonaler Antikörper, die gegen folgende Peptidsequenzen gerichtet sind:
NH₂-V-A-S-T-A-S-D-E-A-R-E-S-S-R-K-S-COOH (SEQ ID NO 1),
NH₂-N-N-H-S-V-E-N-S-S-Q-T-A-S-Q-S-V-COOH (SEQ ID NO 2),
NH₂-G-Q-Y-A-A-T-Q-E-R-S-E-Q-Q-I-S-COOH (SEQ ID NO 3),
NH₂-L-G-I-K-D-S-N-K-Q-I-S-P-E-H-COOH (SEQ ID NO 4) und
NH₂-L-N-M-K-K-T-G-T-D-A-T-K-N-L-N-COOH (SEQ ID NO 5)
bzw. mit zu den genannten Sequenzen korrespondierenden Nukleinsäuresequenzen. Die erfindungsgemäß eingesetzten Antikörper werden mittels Antigenen gewonnen, die das komplette SipC oder Untereinheiten davon darstellen, wobei die Aminosäuresequenz des kompletten SipC nicht verwendet wird.

Als Antigene werden auch die o. g. synthetisch hergestellten Peptide verwendet, die nach Immunisierung von Tieren Antikörper induzieren, die SipC bzw. seine hydrophilen und hydrophoben Untereinheiten erkennen.

Die gewonnenen Antikörper können einzeln oder in einer Kombination in immunchemischen Nachweissystemen verwendet werden.

Das nicht erfindungsgemäße Verfahren zur Gewinnung von mono- und/oder polyklonalen Antikörpern, die spezifisch mit SipC reagieren und durch übliche Immunisierungsverfahren induziert werden, ist dadurch gekennzeichnet, dass man die o. g. Peptide
NH₂-V-A-S-T-A-S-D-E-A-R-E-S-S-R-K-S-COOH (SEQ ID NO 1),
NH₂-N-N-H-S-V-E-N-S-S-Q-T-A-S-Q-S-V-COOH (SEQ ID NO 2),
NH₂-G-Q-Y-A-A-T-Q-E-R-S-E-Q-Q-I-S-COOH (SEQ ID NO 3),
NH₂-L-G-I-K-D-S-N-K-Q-I-S-P-E-H-COOH (SEQ ID NO 4),
NH₂-L-N-M-K-K-T-G-T-D-A-T-K-N-L-N-COOH (SEQ ID NO 5)
oder immunogene Teilpeptide davon als Antigen zur Immunisierung von Vertebraten, insbesondere von Kleinsäugern und Vögeln, verwendet.

Es hat sich als zweckmäßig erwiesen, die freien Peptide vor der Immunisierung an geeignete Trägersubstanzen, vorzugsweise Hämocyanin oder Albumin koppeln.

Polyklonale Antikörper können u. a. unter Einsatz von Hühnern produziert werden.

Gegenstand der Erfindung sind nicht die eingesetzten polyklonalen bzw. monoklonalen Antikörper, die wie beschrieben hergestellt werden.

Alle Reinigungs- und Detektionssysteme für SipC, die zumindest einen der erfindungsgemäßen Antikörper gegen die o. g. Peptide enthalten, sind von der Patentanmeldung umfasst.
Mit den Antikörpern werden immunologische Testkits mit einem oder mehreren Antikörpern zur Diagnose/Bestimmung von Salmonelleninfektionen/-kontaminationen aus Stuhl bzw. unterschiedlichsten Matrizes aufgebaut.
Solche Testkits können auch 2 unterschiedliche Antikörper enthalten (Sandwich-ELISA).
Das Verfahren zur Herstellung eines Testsystems zur Erkennung/Bestimmung von Salmonelleninfektionen/-kontaminationen aus Stuhlproben bzw. unterschiedlichsten Matrizes umfasst die Schritte:
a. Chemische Synthese der identifizierten Peptidsequenzen oder
b. Verdau von SipC durch proteolytische/chemische Spaltung und Nutzung der Spaltprodukte zur Immunisierung von Versuchstieren mit anschließender Gewinnung von Antikörpern aus diesen Tieren bzw. von Zellen aus Lymphknoten/Milz/Blut oder anderen Körperteilen und Fusionierung dieser Zellen mit Myelomzellen,
c. Gewinnung und Aufreinigung der in den Tieren oder in einem von diesen gelegten Ei gebildeten Antikörpern bzw. Isolierung von monoklonalen Antikörpern aus Zellkulturflüssigkeit,
d. Bindung einer Kombination der aufgereinigten Antikörper an einen geeigneten Träger.

Die Spaltung der im Kulturüberstand enthaltenen Proteine wird mittels Bromcyan oder einer oder mehreren Proteasen durchgeführt. Als Proteasen wird bevorzugt eine Kombination von Trypsin und Pepsin verwendet. Wenn die Antikörper durch Immunisierung erzeugt werden, werden die Antikörper aus einem von dem Huhn gelegten Ei aufgereinigt. Weitere Reinigungen der Antikörper können über eine Protein A-Säule erfolgen oder durch anderweitige affinitäts- bzw. gel- oder ionenchromatographische Verfahren oder durch fraktionierte Fällung vorgenommen werden. Die aufgereinigten Antikörper können chemisch oder adsorptiv an feste Träger gebunden werden.

Der Träger ist bevorzugt als Partikel, Membran oder Platte gebildet. Er besteht aus Nitrocellulose-, Celluloseacetat- bzw. PTFE-Membranen oder der Kavität einer Mikrotiterplatte bzw. als plane Platte oder sphärischem Partikel.

Gemäß der Offenbarung besteht das Diagnose-/Bestimmungsverfahren von Salmonelleninfektionen, aus den Schritten
a. Herstellung eines Testsystems,
b. Inkubation des Trägers mit Stuhl/Stuhlanreicherungen/Kot/Kotanreicherungen/ Anreicherungen-Vorkultivierungen sonstiger Substrate,
c. Nachweis der aus den unter b) genannten Untersuchungssubstraten an den Träger gebundenen Proteine mit Hilfe von Detektionsantikörpern.

Bei den sonstigen Substraten handelt es sich um Schweine und Teile vom Schwein u. a. Säugetieren/ Vögeln (Fleisch, Blut, Organe) Umweltproben (Kot, Tupferproben, Kratzschwammproben, Stiefel-/Sockenproben, Stallstaub) Lebens- und Futtermittel. Bei dem Verfahren können sowohl die Detektionsantikörper als auch die aufgereinigten Antikörper als Sekundärantikörper gebildet sein.

In anderen Fällen werden auch kreuzreaktive Antikörper und/oder eine Kombination von Antikörpern unterschiedlicher Herkunft verwendet.

Das Verfahren ist dadurch gekennzeichnet, dass ein immunchemisches Nachweisverfahren verwendet wird, wobei es sich um einen ELISA, einen Streifen-oder Spotassay handeln kann. Die Detektionsantikörper sind bevorzugt markiert, wobei Biotin, Peroxidase, Gold oder Fluoreszenzfarbstoffe in Betracht kommen. Wo es sinnvoll ist, können Peroxidase-markiertes Streptavidin und/oder Peroxidasesubstrat verwendet werden. Als Peroxidasesubstrat dient bevorzugt TMB. Die Umsetzung des Peroxidase-Substrats wird durch ein optisch sichtbares Produkt nachgewiesen.

### Ausführungsb ispi I 1 - Herstellung spezifischer anti-Peptid-Antikörper, die gegen definierte Abschnitte des SipC-Proteins gerichtet sind (nicht erfindungsgemäß).

Mittels Festphasensynthese nach Merrifield werden die Peptide mit den Aminosäuresequenzen NH₂-V-A-S-T-A-S-D-E-A-R-E-S-S-R-K-S-COOH (SEQ ID NO 1), NH₂-N-N-H-S-V-E-N-S-S-Q-T-A-S-Q-S-V-COOH (SEQ ID NO 2), NH₂-G-Q-Y-A-A-T-Q-E-R-S-E-Q-Q-I-S-COOH (SEQ ID NO 3), NH₂-L-G-I-K-D-S-N-K-Q-I-S-P-E-H-COOH (SEQ ID NO 4) und NH₂-L-N-M-K-K-T-G-T-D-A-T-K-N-L-N-COOH (SEQ ID NO 5) synthetisiert. Die Peptide werden mit bekannten Verfahren an Napfschneckenhämocyanin (KLH) gekoppelt (1 mg Peptid/mg KLH). Je 300 µg dieses Konjugates werden unter Zusatz von Freundschen Adjuvants für die Immunisierung von Kaninchen bzw. Hühnern verwendet. Nach 3maliger Immunisierung werden die Tiere entblutet. Nach der Gewinnung des Serums wird die Spezifität der Antiseren in einem ELISA getestet. Dazu wird freies Peptid an die Oberfläche der Kavitäten von Mikrotiterplatten adsorbiert. Nach Inkubation der Kavitäten mit den Antiseren werden diese gründlich gewaschen. Unter Verwendung von Antikaninchen- bzw. Antihuhn-POD-Konjugat und TMB als Substrat wird in üblicher Weise die Antigen-Antikörperreaktion detektiert. Jedes Antiserum reagiert nur mit dem homologen Peptid.

### Ausführungsbeispiel 2 - Nachweis der Spezifität der Antikörper (nicht erfindungsgemäß)

Die SipC-Spezifität kann im Westernblot nachgewiesen werden. Dazu wird grob- bzw. hochgereinigtes SipC aus Kulturüberständen von Salmonella ssp. mittels Polyacrylamidgelelektrophorese entsprechend ihrer relativen Molmasse von begleitenden Verunreinigungen getrennt. Die Proteinzonen aus dem Gel werden mit Hilfe einer "Semi-dry-blotting"-Apparatur auf Nitrozellulose übertragen. Nach Absättigung der freien Bindungsstellen der Membran mit resuspendierter Trockenmagermilch werden die Membranen mit den 1:500 verdünnten anti-Peptid-Antiseren inkubiert. Nach intensivem Waschen der Membranen zur Entfernung aller unspezifisch gebundenen Antikörper werden die Membranen mit Phosphatasemarkierten anti-Spezies-Antikörpern inkubiert.

Die spezifisch gebundenen sekundären Antikörper, die nach dem Waschen auf der Membran verbleiben, werden nach Zugabe des Substrates sichtbar gemacht. Dabei zeigt sich, dass in den verwendeten Proben ausschließlich SipC nachgewiesen wird.

### Ausführungsbeispi I 3 - Bestimmung von SipC in Kulturüberständen von Salmonella spp. unter Verwendung der Antikörper in einem ELISA (nicht erfindungsgemäß)

Das SipC in Kulturüberständen von Salmonella ssp. wird in einem Festphasen-Enzymimmunoassay, der auf der Sandwichtechnik basiert, bestimmt. Ein polyklonaler Antikörper, der gegen Epitope des SipC gerichtet ist, wird in einem Karbonat/Bikarbonat-Puffergemisch, pH 9,6 gelöst und in die Wells einer Mikrotiterplatte gegeben. Nach der Inkubation bei 4° C über 12 h werden die nicht gebundenen Antikörper durch Waschen mir PBS entfernt. Die noch freien Bindungsstellen des Trägermaterials werden durch einen PBS-Puffer, der Rinderserumalbumin und Tween 20 enthält, geblockt. Das Blocken findet bei Raumtemperatur über 90 min statt. Nach dem Waschen werden die in PBS verdünnten Kulturüberstände in die Wells pipettiert. Die 60-minütige Inkubation bei Raumtemperatur wird durch das Waschen beendet. Ein zweiter SipC spezifischer polyklonaler Antikörper, der mit Biotin konjugiert ist, wird zu dem an den ersten Antikörper gebundenem SipC gegeben.

Nach einer Inkubation von 30 min und dem Waschprozess wird der biotinmarkierte Antikörper mit Peroxidase-markiertem Streptavidin nachgewiesen. Durch den letzten Waschschritt erfolgt die Beseitigung des nicht gebundenen Streptavidins. Anschließend wird TMB als Substrat für die Peroxidase dazugegeben und nach einer definierten Zeit wird die Farbreaktion durch Zugabe von HCl abgestoppt. Gemessen wird die Änderung der optischen Dichte. Die Intensität der Farbreaktion ist proportional der SipC-Konzentration in der Probe.

### Ausführungsbeispiel 4 - Bestimmung des SipC in Stuhl/Kot unter Verwendung der Antikörper (nicht erfindungsgemäß)

Das SipC im Stuhl/Kot wird derart bestimmt, dass zunächst eine Voranreicherung der Salmonellen über 4 bis 8 h in Peptonwasser vorgenommen wird. Danach wird das SipC mit einem Festphasen-ELISA, der auf der Sandwichtechnik basiert, bestimmt. Dazu werden einzelne oder ein entsprechendes Gemisch mehrerer der Antikörper in einem Karbonat/Bikarbonat-Puffergemisch, pH 9,6 gelöst und in die Wells einer Mikrotiterplatte gegeben. Nach Inkubation bei 4° C werden die nicht gebundenen Antikörper durch Waschen mit PBS entfernt. Die noch freien Bindungsstellen des Trägermaterials werden durch einen PBS-Puffer, der Rinderserumalbumin und Tween 20 enthält, geblockt. Das Blocken findet bei Raumtemperatur über 90 min statt. Nach dem Waschen werden die in PBS verdünnten Stuhlproben in die Wells pipettiert. Die 60-minütige Inkubation bei Raumtemperatur wird durch Waschen beendet. Als Detektionsantikörper werden einzelne oder ein entsprechendes Gemisch aus mehreren Antikörpern verwendet, die mit Biotin konjugiert wurden. Nach einer Inkubation von 30 min und dem Waschprozess wird der biotinmarkierte Antikörper mit Peroxidase-markiertem Streptavidin nachgewiesen. Durch den letzten Waschschritt erfolgt die Beseitigung des nicht gebundenen Streptavidins. Anschließend wird mit TMB als Substrat die Peroxidasekonzentration bestimmt. Nach Zugabe von HCl zur Beendigung der Enzymreaktion wird die Änderung der optischen Dichte gemessen. Die Intensität der Farbreaktion ist proportional der SipC-Konzentration in der Probe.

**Ausführungsbeispiel 5** - Bestimmung des SipC aus unterschiedlichem Untersuchungsmaterial wie:
- Schweinen und Teilen vom Schwein u. a. Säugetieren/ Vögeln (Fleisch, Blut, Organe)
- Umweltproben (Kot, Tupferproben, Kratzschwammproben, Stiefel-/Sockenproben, Stallstaub)
- Lebens- und Futtermittel
unter Verwendung der erfindungsgemäßen Antikörper. wird derart bestimmt, dass zunächst eine Voranreicherung der *Salmonella spp.* stattfindet. Eine definierte Menge des Untersuchungsmaterials wird zur Induzierung der Sekretion in gepuffertem Peptonwasser oder anderen Voranreicherungsmedien 4 - 8 h bei 37° C bebrütet. Ohne weitere selektive Anreicherung und Isolierung wird der Überstand zentrifugiert und das enthaltene SipC mit einem Festphasen-ELISA, der auf der Sandwichtechnik basiert, bestimmt. Dazu werden einzelne oder ein entsprechendes Gemisch mehrerer der Antikörper in einem Karbonat/Bikarbonat-Puffergemisch, pH 9,6 oder anderem Puffer gelöst und in die Wells einer Mikrotiterplatte gegeben. Nach Inkubation bei 4° C werden die nicht gebundenen Antikörper durch Waschen mit PBS entfernt. Die noch freien Bindungsstellen des Trägermaterials werden durch einen PBS-Puffer, der Rinderserumalbumin und Tween 20 enthält, geblockt. Das Blocken findet bei Raumtemperatur über 90 min statt. Nach dem Waschen werden die in PBS verdünnten Proben in die Wells pipettiert. Die 60-minütige Inkubation bei Raumtemperatur wird die Reaktion durch Waschen beendet. Als Detektionsantikörper werden einzelne oder ein entsprechendes Gemisch aus mehreren erfindungsgemäßen Antikörpern verwendet, die mit Biotin konjugiert wurden. Nach einer Inkubation von 30 min und dem Waschprozess wird der biotinmarkierte Antikörper mit Peroxidase-markiertem Streptavidin nachgewiesen. Durch den letzten Waschschritt erfolgt die Beseitigung des nicht gebundenen Streptavidins. Anschließend wird mit TMB als Substrat die Peroxidasekonzentration bestimmt. Nach Zugabe von HCl zur Beendigung der Enzymreaktion wird die Änderung der optischen Dichte gemessen. Die Intensität der Farbreaktion ist proportional der SipC-Konzentration in der Probe.

**Ausführungsbeispiel 6** - Bestimmung des SipC aus unterschiedlichem Untersuchungsmaterial wie
- Schweinen und Teilen vom Schwein u. a. Säugetieren/ Vögeln (Fleisch, Blut, Organe)
- Umweltproben (Kot, Tupferproben, Kratzschwammproben, Stiefel-/Sockenproben, Stallstaub)
- Lebens- und Futtermittel
- Stuhl/Kot
in einem Streifenschnelltest (direkter Assay)

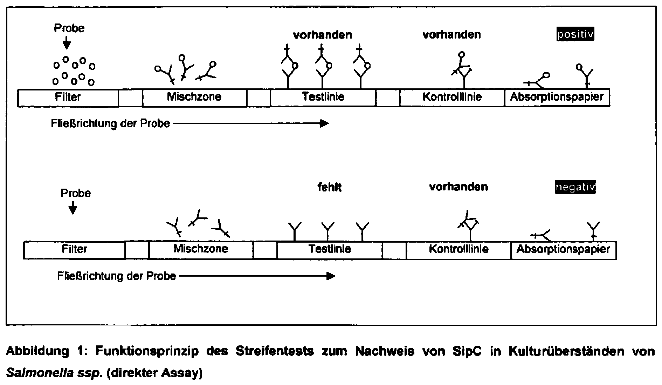

### Testschema: siehe Abbildung 1

Das SipC in Kulturüberständen von *Salmonella ssp.* wird in einem qualitativen immunchromatographischen Streifentest bestimmt. Der Test basiert auf einer spezifischen Reaktion von Gold-konjugierten anti-SipC-Antikörpern mit freiem SipC in der Probe. Die Testvorrichtung besteht aus einer Plastikbasis mit aufgelagerter Nitrozellulosemembran (Sigma Aldrich). Anti-SipC-Antikörper und gereinigte Anti-Spezies-Antikörper sind auf zwei Linien (Testlinie und Kontrolllinie) immobilisiert. An die gegen Epitope des Proteins SipC gerichteten aufgereinigten Antikörper wurden Goldpartikel gebunden (40 nm, British Biocell International, Cardiff, GB). Die konjugierten Antikörper wurden auf ein Konjugatkissen gegeben (Arista Biologicals, Allentown, PA, USA). Dieses Konjugatkissen überlappt sich mit der Nitrozellulosemembran. Die Probe wird auf das Probenfeld aufgetragen. Enthält die Probe SipC, bindet dieses an die Gold-konjugierten Antikörper. Nach Zugabe von Probenpuffer wandern die Probe und die Antikörper durch Kapillarkräfte an der Nitrozellulosemembran entlang. Die durch Pufferzugabe resuspendierten Anti-SipC-Antikörper wandern in Richtung Testlinie und Kontrolllinie. Ist SipC von den Gold-markierten Antikörpern gebunden und bindet das Protein mit einer anderen immunogenen Determinante an die immobilisierten Antikörper auf der Testlinie, entsteht an dieser Stelle eine rote Linie. An der Kontrolllinie wird eine rote Bande sichtbar, sobald die mit dem Probenpuffer gewanderten Antikörper von den anti-Spezies-Antikörpern auf der Kontrolllinie gebunden werden. Ist in der Probe kein SipC vorhanden, werden die Gold-markierten anti-SipC-Antikörper nicht über das Protein SipC an die Antikörper auf der Testlinie immobilisiert und es kommt lediglich zur Ausbildung einer roter Bande an der Kontrolllinie. Die Ausbildung der Farbreaktion ist für beide Linien nach etwa 10 - 15 Minuten abgeschlossen.

### Ausführungsbeispiel 7 - Bestimmung von SipC in Kulturen von Salmonella ssp. mittels Fluoreszenz In-situ Hybridisation (FISH) unter Verwendung von markierten Sonden gegen Abschnitte der Nukleinsäuresequenz des SipC (nicht erfindungsgemäß)

Die Methode basiert auf der Hybridisierung von Oligonukleotiden, die mit einem Fluorochrom (z.B. CY3) markiert sind, an ihre komplementäre Sequenz auf dem Zielmolekül (DNA/RNA). Die Oligonukleotide bestehen in der Regel aus 15-25 Nucleotiden, haben ein ausgeglichenes Verhältnis von A/T zu G/C, haben einen Schmelzpunkt zwischen 50° C und 70° C, tragen in sich keine komplementären Bereiche und tragen G/C-Basen an ihren Enden. Die Nukleinsäuresonden können an extrahierte Nukleinsäuren binden oder zur "Ganz-Zell-Präparation" genutzt werden. Für letztere Methode ist es notwendig die Zellen zu permeabilisieren. Für Gramnegative Bakterien geschieht dies in der Regel durch die Fixierung der Proben mit (Para)-Formaldehyd. Die Hybridisierung der fluorochrommarkierten Sonde an die Zielsequenz ist abhängig von der Zugänglichkeit der Ziel-DNA, den Charakteristika der Sonde (Länge, Schmelztemperatur, A/T:C/G-Verhältnis) und den Hybridisierungsbedingungen (Puffer, Temperatur, Inkubationszeit). Da DNA in der Regel doppelsträngig vorliegt, muss diese vorher getrennt werden. Dies geschieht normalerweise durch pH-Wert-Verschiebung oder durch Temperaturerhöhung. Bei der Hitzedenaturierung wird die Schmelztemperatur durch Zugabe von Formaldehyd abgesenkt. Die Denaturierung kann somit bereits bei Temperaturen von etwa 70 °C erreicht werden. Die sogenannte Stringenz (Bindungsstärke bei bestimmter Temperatur) der Sonde an die Zielsequenz ist abhängig von der Salz-, Formaldehyd-und Probenkonzentration. Bei gleich bleibender Hybridisierungstemperatur führt steigende Formaldehydkonzentration und sinkende Salzkonzentration zu einer höheren Stringenz. Nicht gebundene Oligonucleotide werden in einem Waschschritt aus den Zellen ausgewaschen. So lassen sich Zellen, die die Sonde nicht spezifisch gebunden haben von den Zielzellen unterscheiden. Zellen, die die Sonde nicht gebunden haben weisen kein FISH-Fluoreszenzsignal auf, können aber durch DAPI-Gegenfärbung markiert werden. Während des Waschschrittes ist die Temperatur gleich der Inkubationstemperatur. Lediglich die Salzkonzentration des Waschpuffers ist geringer als die des Hybridisierungspuffers. So wird die Stringenz der gebundenen Proben erhöht.

Folgende Sonden wurden beispielhaft erfolgreich getestet:
**5'-GGATTGCGAAGCTGTCTGTGAACTATTCTC-3'** (SEQ ID NO 8)
**5'-CGTCGCATCGGTACC-3'** (SEQ ID NO 9)
**5'-GAGATTTGTTTATTACTG-3'** (SEQ ID NO 10)
**5'-GAACGTTCCTGAGTAGCGGC-3'** (SEQ ID NO 11)
**5'-CACGGGCTTCGTCCGATGCGGTGCTGGC-3'** (SEQ ID NO 12)
Am 5'-Ende sind die Sonden mit dem Fluoreszenzfarbstoff Cy3 markiert.

Nach Vorkultivierung der zu untersuchenden Proben werden diese mit Formaldehyd (Endkonzentration von 2 %) fixiert und die Bakterienzellen somit permeabilisiert. Nach 30-minütiger Inkubation bei 4° C werden die Ansätze auf 75° C erhitzt, um die doppelsträngige DNA zu denaturieren. Die Sonden werden in Hybridisierungspuffer verdünnt und zu den Ansätzen gegeben. Die Hybridisierung erfolgt bei einer Temperatur von 45 ± 1° C. Nach 3 h Inkubation werden die Ansätze bei 5000 g⁻¹ abzentrifugiert und das Pellet anschließend in Ethanol/PBS-Gemisch aufgenommen (Verhältnis 1+1). Dieser Waschschritt wird zweimal wiederholt, um die nicht gebundenen Sonden aus den Zellen auszuwaschen. Die resuspendierten Proben werden auf einen Objektträger aufgebracht und bei einer Temperatur von 45 ± 1° C getrocknet. Nach dem Trocknen werden der Objektträger für jeweils 2 min in 50 %igem, 80 %igem und 96 %igem Ethanol entwässert und danach luftgetrocknet. Nach dem Trocknen können die Objektträger unter Verwendung eines Fluoreszenzmikroskops ausgewertet werden.

### Ausführungsb ispi I 8 - Bestimmung von SipC in Kulturen von Salmonella ssp. mittels Nukleinsäureamplifikationstechniken (PCR) unter Verwendung von spezifischen Primern für die Nukleinsäuresequenz des SipC (nicht erfindungsgemäß)

Allgemein ist die PCR eine Methode, um einen definierten Teile eines DNA-Stranges *in vitro* zu vervielfältigen (amplifizieren). Die PCR stellt eines der sichersten Nachweisverfahren für Bakterien (und andere Organismen) auf DNA-Ebene dar, benötigt aber aufgrund seiner Selektivität gesicherte Informationen zu Bakterientyp und -klasse. Die Wahl der Primer sollte so gestaltet sein, dass ein DNA-Fragment amplifiziert wird, das spezifisch für den zu identifizierenden Organismentyp ist. So wurden zur Detektion von Salmonellen Primerpaare gewählt, die jeweils komplementär zu einem Strang der DNA-Sequenz des SipC-Proteins sind.

### Folgende Primerpaare wurden beispielhaft erfolgreich getestet:

| **Richtung** | **5'-Pos** | **Primer Sequ nz (5'->3')** | **Produktlänge (bp)** |
|---|---|---|---|
| | | | |
| Forward | 25 | ATCCCGCCGCTTATTTAAATAATCATTCTG (SEQ ID NO 13) | 1186 |
| Reverse | 1210 | CGATAGCAGCGAGTGCGG (SEQ ID NO 14) | |
| | | | |
| Forward | 25 | ATCCCGCCGCTTATTTAAATAATCATTCTG (SEQ ID NO 15) | 1176 |
| Reverse | 1200 | GAGTGCGGATGCTTTCGACTGG (SEQ ID NO 16) | |
| | | | |
| Forward | 62 | TAGTTCACAGACAGCTTCGCAATCC (SEQ ID NO 17) | 1117 |
| Reverse | 1178 | TTAATGCTCTCCATTGTTTTCAGCATTTCC (SEQ ID NO 18) | |
| | | | |
| Forward | 68 | ACAGACAGCTTCGCAATCCGTTAG (SEQ ID NO 19) | 1108 |
| Reverse | 1175 | ATGCTCTCCATTGTTTTCAGCATTTCCTG (SEQ ID NO 20) | |
| | | | |
| Forward | 68 | ACAGACAGCTTCGCAATCCGTTAG (SEQ ID NO 21) | 1152 |
| Reverse | 1219 | TATTGCCTGCGATAGCAGCGAG (SEQ ID NO 22) | |

Die Erfindung stellt ein Verfahren gemäß Anspruch 1 zur Verfügung, welches einen schnellen Nachweis von Salmonelleninfektionen ermöglicht. Während die bekannten Verfahren 3-5 Tage für eine gesicherte Aussage benötigen, führt das erfindungsgemäße Verfahren gemäß Anspruch 1 bereits nach ca. 10 Stunden zum Ergebnis.

Ein weiterer Vorteil liegt in der universellen Anwendbarkeit, alle wichtigen Serovare werden zuverlässig erfasst.

## Patentansprüche

1. Diagnostisches Verfahren zur Erkennung einer Salmonelleninfektion/- kontamination in Exkreten von Patienten/Tieren, Schlachtkörpern, Eiern und Lebens-/Futtermitteln durch den Nachweis des von den Salmonellen ausgeschiedenen SipC Proteins, **dadurch gekennzeichnet, dass** die Bestimmung von SipC durch die Verwendung von monoklonalen Antikörpern erfolgt, die gegen folgende Peptidsequenzen von SipC gerichtet sind:
• NH₂-V-A-S-T-A-S-D-E-A-R-E-S-S-R-K-S-COOH (SEQ ID NO 1)
• NH₂-N-N-H-S-V-E-N-S-S-Q-T-A-S-Q-S-V-COOH (SEQ ID NO 2),
• NH₂-G-Q-Y-A-A-T-Q-E-R-S-E-Q-Q-I-S-COOH (SEQ ID NO 3),
• NH₂-L-G-I-K-D-S-N-K-Q-I-S-P-E-H-COOH (SEQ ID NO 4) und
• NH₂-L-N-M-K-K-T-G-T-D-A-T-K-N-L-N-COOH (SEQ ID NO 5).

2. Verfahren nach Anspruch 1, **dadurch gekennzeichnet, dass** die Antikörper einzeln oder in einer Kombination in immunchemischen Nachweissystemen verwendet werden.

3. Diagnostisches Verfahren nach Anspruch 1 - 2, **dadurch gekennzeichnet, dass** zwei unterschiedliche Antikörper als Sandwich-ELISA eingesetzt werden.

## Claims

1. A diagnostic method for detecting a salmonella infection/contamination in excretions of patients/animals, carcasses, eggs and food/feed by the detection of the SipC protein excreted by the salmonellae, **characterized in that** SipC is determined by the use of monoclonal antibodies directed against the following peptide sequences of SipC:
• NH₂-V-A-S-T-A-S-D-E-A-R-E-S-S-R-K-S-COOH (SEQ ID NO 1)
• NH₂-N-N-H-S-V-E-N-S-S-Q-T-A-S-Q-S-V-COOH (SEQ ID NO 2),
• NH₂-G-Q-Y-A-A-T-Q-E-R-S-E-Q-Q-I-S-COOH (SEQ ID NO 3),
• NH₂-L-G-I-K-D-S-N-K-Q-I-S-P-E-H-COOH (SEQ ID NO 4) und
• NH₂-L-N-M-K-K-T-G-T-D-A-T-K-N-L-N-COOH (SEQ ID NO 5).

2. Method according to claim 1, **characterized in that** the antibodies are used individually or in combination in immunochemical detection systems.

3. A diagnostic method according to Claim 1 - 2, **characterized in that** two different antibodies are used as sandwich ELISA.

## Revendications

1. Procédé de diagnostic pour le dépistage d'une infection/contamination par des salmonelles dans des excrétions de patients/d'animaux, de carcasses, d'oeufs et de denrées alimentaires/d'aliments pour animaux par la détection de la protéine SipC excrétée par les salmonelles, **caractérisé par le fait que** la détermination de SipC s'opère par l'utilisation d'anticorps monoclonaux qui sont dirigés contre les séquences peptidiques de SipC suivantes :
• NH₂-V-A-S-T-A-S-D-E-A-R-E-S-S-R-K-S-COOH (SEQ ID NO 1)
• NH₂-N-N-H-S-V-E-N-S-S-Q-T-A-S-Q-S-V-COOH (SEQ ID NO 2),
• NH₂-G-Q-Y-A-A-T-Q-E-R-S-E-Q-Q-I-S-COOH (SEQ ID NO 3),
• NH₂-L-G-I-K-D-S-N-K-Q-I-S-P-E-H-COOH (SEQ ID NO 4) et
• NH₂-L-N-M-K-K-T-G-T-D-A-T-K-N-L-N-COOH (SEQ ID NO 5).

2. Procédé conformément à la revendication n°1, **caractérisé par le fait que** les anticorps sont utilisés séparément ou dans une combinaison dans des systèmes de détection immunochimiques.

3. Procédé de diagnostic conformément à la revendication n°1 à n°2, **caractérisé par le fait que** deux anticorps différents sont utilisés comme ELISA en sandwich.
